# EUROPEAN PATENT APPLICATION

(11) **EP 4 792 742 A1**
(43) Date of publication of application: **19.08.2026**
(21) Application number: 25157679.9
(22) Date of filing: 13.02.2025
(51) Int. Cl.: A61B 6/00, A61B 34/20, A61B 17/00, A61B 90/00

(54) **TECHNIQUE FOR IMPROVING USE OF A MEDICAL IMAGING DEVICE AND AN OPTICAL TRACKING SYSTEM**

(71) Applicant: Stryker European Operations Limited, Carrigtwohill, Co. Cork T45 HX08 (IE)
(72) Inventor: Kaul, Thorben, 79106 Freiburg (DE); Pfeil, Antoine, 67530 Boersch (FR); Berndt, Marc, 8049 Zürich (CH)
(74) Representative: Schott, Jakob Valentin

(57) **Abstract**

Disclosed is a method for improving use of a medical imaging device and an optical tracking system, the method comprising: obtaining a current pose of a medical imaging device relative to an optical tracking system; obtaining a predicted movement that the medical imaging device will undergo for acquiring a medical image of a patient; determining a predicted movement path that is associated with the medical imaging device; and based on the predicted movement path, triggering display of a visualization that is configured to guide a user in repositioning the optical tracking system and the medical imaging device relative to one another such the medical imaging device can be tracked via the optical tracking system over at least a predefined minimum portion of the predicted movement path.

## Description

### Technical Field

The present disclosure generally relates to a method for improving use of a medical imaging device and an optical tracking system. A surgical navigation system, a computer program and a carrier are also disclosed herein.

### Background

In many surgical scenarios, surgeons nowadays rely on computer guidance to navigate medical instruments, implants or the like relative to a patient's body. To this end, medical images of the patient's body can be registered to the patient's body before surgical navigation is started. In some scenarios, a patient tracker is attached to the patient's body. To register the medical images to the patient's body, a transformation between an image coordinate system of the medical images and another coordinate system associated with the patient tracker can be used. If the pose of the medical imaging device during capture of the medical images is known in a tracking coordinate system, a transformation between the image coordinate system of the captured medical images and the tracking coordinate system can be derived. Once the pose of the patient's body is also known in said tracking coordinate system, the transformation between the image coordinate system of the medical images and the other coordinate system (e.g., the tracking coordinate system) associated with the patient tracker can be determined. This, in turn, enables navigating components that are tracked in the tracking coordinate system, for example by providing a visualization of the medical patient images with an overlay of the navigated components in their respective tracked poses.

The above and other approaches used in modern surgical procedures require the determination of a spatial pose of the medical imaging device, or even require a tracking of the medical imaging device over time. An optical tracking system can be used for this purpose. However, in some scenarios, the medical imaging device may be placed relative to the optical tracking system in such a manner that it cannot be tracked at all, or such that it can only be tracked over unacceptably small portions of movement of the medical imaging device during image acquisition. Still further, current approaches typically employ rather large mechanical trackers with multiple tracking markers that must be attached to the medical imaging device for tracking the same via the optical tracking system, which trackers may change their attachment pose relative to the imaging device over time and due to their size may require the optical tracking system to have a large field of view. Therefore, current approaches for localizing and/or tracking medical imaging devices leave room for improvements.

### Summary

There is a need for a technique that solves one or more of the aforementioned or other problems.

According to a first aspect, a method for improving use of a medical imaging device and an optical tracking system is provided. The method is performed by at least one processor and comprises: obtaining pose data indicative of a current pose of a medical imaging device relative to an optical tracking system; obtaining movement data indicative of a predicted movement that the medical imaging device will undergo for acquiring a medical image of a patient; determining, based on the pose data and the movement data, and relative to the optical tracking system, a predicted movement path that is associated with the medical imaging device; and based on the predicted movement path, triggering display of a visualization that is configured to guide a user in repositioning the optical tracking system and the medical imaging device relative to one another such the medical imaging device can be tracked via the optical tracking system over at least a predefined minimum portion of the predicted movement path.

The term "pose" as used herein means at least one of position and orientation. Each pose may be defined in six degrees of freedom.

The method may further comprise: obtaining viewing data indicative of a current field of view of the optical tracking system; and determining, based on the predicted movement path and the viewing data, at least one first portion of the predicted movement path that extends through the field of view of the optical tracking system and/or at least one second portion of the predicted movement path that extends outside the field of view of the optical tracking system, wherein the visualization is determined based on one or more of said determined portion(s) of the predicted movement path.

The method may further comprise: obtaining object data indicative of a current pose of an object arranged within the field of view of the medical imaging device; and determining, based on the predicted movement path and the object data, at least one third portion of the predicted movement path that is hidden to the optical tracking system by the object, wherein the visualization is determined based on one or more of said determined portion(s) of the predicted movement path.

The method may further comprise: determining a representation of said one or more determined portion(s) relative to the field of view of the optical tracking system, wherein the visualization includes said representation.

The representation may be determined such that it complies with a viewing direction of a camera of the optical tracking system.

The representation may be determined as having an optical property that depends on a proportion between {i} a total size of all of the at least one first portion or a continuous section thereof and {ii} a total size of all of the at least one second portion or a continuous section thereof.

The optical property may be selected from one of a predefined set of optical properties, each of the predefined set of optical properties being associated with a different range of the proportion.

The predefined set of optical properties may consist of a plurality of different colors and/or a plurality of different patterns.

The method may further comprise: obtaining an image or video stream acquired by the camera of the optical tracking system, wherein the visualization comprises an overlay of the determined representation over the image or video stream.

The image may be a color image. The video stream may be a color video stream.

The pose data may be indicative of a pose of a tracker that is mounted to the medical imaging device and has a predefined relative pose thereto.

The predicted movement path may describe a path along which a tracker, mounted to the medical imaging device and having a predefined relative pose thereto, moves when the medical imaging device undergoes its predicted movement for acquiring a medical image of a patient.

The tracker may comprise a single passive optical tracking marker.

The method may further comprise: obtaining first tracking data indicative of a plurality of first positions of the tracker at different points in time during a first movement of the medical imaging device relative to the optical tracking system; and based on the plurality of first positions, determining the current pose of the medical imaging device relative to the optical tracking system to thereby obtain the pose data.

Based on the plurality of first positions, a center of rotation of the medical imaging device and a current position of the tracker may be determined, and the current pose of the medical imaging device relative to the optical tracking system may be determined based on the center of rotation of the medical imaging device and the current position of the tracker.

The method may further comprise: obtaining updated pose data indicative of a repositioned pose of the medical imaging device relative to the optical tracking system; updating the predicted movement path based on the updated pose data; and based on the updated predicted movement path, updating the visualization that is triggered to be displayed.

The method may further comprise: obtaining second tracking data indicative of a plurality of second positions of the tracker at different points in time during a second movement of the medical imaging device, for acquiring a medical image, relative to the optical tracking system; based on the plurality of second positions, determining a center of rotation of the medical imaging device; and registering medical image data associated with the second movement of the medical imaging device, based on the determined center of rotation of the medical imaging device.

The method may further comprise: using the registered medical image data to provide a surgical navigation view for a user.

The method may be referred to as computer-implemented method. The method in one variant does not comprise a surgical step, in particular no substantial interaction with the body of a living human or animal.

According to a second aspect, a surgical navigation system is provided. The surgical navigation system comprises at least one processor, the at least one processor being configured to perform the method of the first aspect. The surgical navigation system may further comprise at least one of the following entities: a display configured to display the visualization; the optical tracking system; the medical imaging device; the tracker.

According to a third aspect, a computer program is provided. The computer program comprises instructions which, when executed by at least one processor, cause the at least one processor to perform the method of the first aspect. The computer program is optionally carried by at least one carrier such as a data stream, a memory or a non-transitory computer storage medium.

According to a fourth aspect, a carrier is provided. The carrier carries the computer program of the third aspect. The carrier may be a data stream, a memory or a non-transitory computer storage medium.

### Brief Description of the Drawings

Further details, advantages and aspects of the present disclosure will become apparent from the following embodiments taken in conjunction with the drawings, wherein:
- Fig. 1: shows an exemplary surgical navigation system in accordance with the present disclosure;
- Fig. 2: shows a flowchart of an exemplary method in accordance with the present disclosure
- Fig. 3: shows an illustration of an exemplary movement path and center of rotation of a medical imaging device in accordance with the present disclosure;
- Fig. 4: shows a first exemplary visualization in accordance with the present disclosure;
- Fig. 5: shows a second exemplary visualization in accordance with the present disclosure; and
- Fig. 6: shows a third exemplary visualization in accordance with the present disclosure.

### Detailed Description

In the following description, exemplary embodiments will be explained with reference to the drawings. Unless indicated otherwise, the reference signs used in the following denote the same or similar structural or functional features.

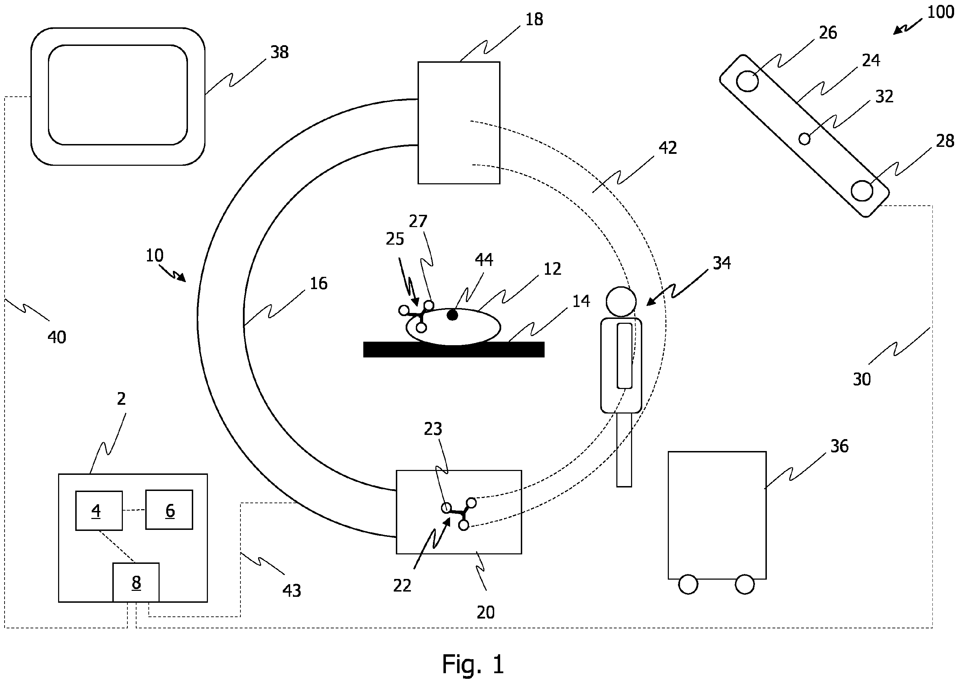

Fig. 1 shows an exemplary surgical navigation system 100. The system 100 comprises a computing unit 2 including a processor 4 communicatively coupled to a memory 6 and an interface 8 configured for wire-bound or wireless communication. The memory 6 stores instructions that, when executed by the processor 4, cause the processor 4 to perform the method disclosed herein.

The system 100 further comprises a medical imaging device 10 configured to acquire medical images of a patient's body 12 which, in the illustrated example, is positioned on a patient couch 14. An optical tracker 25 with one or more optical makers 27 is arranged in a fixed spatial pose relative the patient's body 12. These markers 27 can be localized by an optical tracking system 24, which is also part of the system 100. The medical imaging device 10 is configured to move when acquiring a patient scan of the patient's body 12. In the illustrated example, the medical imaging device 10 is a C-arm scanner comprising a C-arm 16 with an X-ray radiation source 18 and an X-ray detector 20 on opposing ends thereof. For acquiring a CT patient scan, the C-arm 16 rotates around a center of rotation 44, also referred to as isocenter.

An optical tracker 22 is attached to the medical imaging device, for example to the detector 20 or the X-ray source 18. The optical tracker 22 comprises one or more optical markers 23 that can be localized by the optical tracking system 24. In one example, the optical tracker consists of a single optical marker 23 that is for example formed as a sticker and directly attached to the medical imaging device 10. The optical tracking system 24 may comprise two spaced-apart cameras 26, 28 that form a stereo camera. These two cameras may be configured to capture infrared images. The optical tracking system 24 may comprise a camera 32 configured to capture images of humanly visible wavelengths, for example a red-green-blue, RGB, camera. The acquired images can be transmitted via a wired or wireless connection 30 to the computing unit 2, where the processor 4 may localize the optical tracker 22 and other entities such as clinical personnel 34, a clinical cart 36 etc. based on the acquired images.

The system 100 further comprises a display 38 configured to display a visualization as described herein below. The display is communicatively coupled to the computing unit 2 via a wired or wireless connection 40.

When acquiring a patient scan, or when repositioning the C-arm 16 relative to the patient's body 12, the C-arm 16 rotates around the patient's body 12. To this end, movement control instructions may be sent to the medical imaging device 10 via the communication connection 43 and/or medical image data may be sent from the medical imaging device 10 via said connection 43. The optical tracker 22 in this case also moves in space, in particular along a movement path 42. Depending on the relative positioning of the C-arm 16 and the optical tracking system 24, the optical tracking system 24 may be able to capture only part of the movement path 42, capture the entire movement path 42, or capture no part of the movement path 42. It is also possible for the clinical personnel 34 or the cart 36 to shield part of the movement path 42 from the view of the tracking system 24.

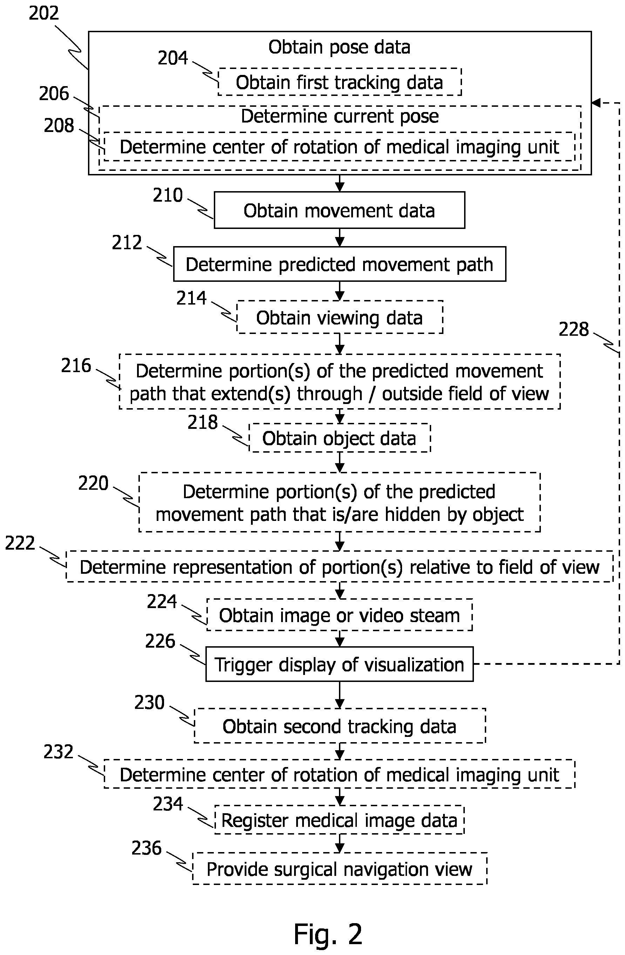

Referring to Fig. 2, a method in accordance with the present disclosure is shown. This method may be performed by the system 100, in particular by the processor 2. The method can be said to be generally intended for improving use of the medical imaging device 10 and the optical tracking system 24. Optional method steps are indicated with dashed lines. It is to be understood that steps may be combined. It is also possible to change the sequence of the method steps compared with the order exemplarily shown in Fig. 2. Although the method will now be described with further reference to Fig. 1, it is to be understood that it may be performed by another processor of another system as well. That is, references below to the components of the system 100 as shown in Fig. 1 are to be interpreted as preferred examples. For instance, instead of the "medical imaging device 10", any other medical imaging device may be used, and the same applies to the other components and beings shown in Fig. 1.

At 202, pose data is obtained. The pose data is indicative of a current pose of the medical imaging device 10 relative to the optical tracking system 24. As mentioned above, the medical imaging device 10 may be a CT scanner, in particular a C-arm configured to acquire a CT scan of a patient's body. The pose data may be indicative of a pose of the tracker 22 that is mounted to the medical imaging device 10 and has a predefined relative pose thereto. The pose of the medical imaging device 10 may be defined in a tracking coordinate system of the optical tracking system 24. The pose of the medical imaging device 10 may also be defined in another coordinate system that has a known spatial relationship to the tracking coordinate system, for example a patient tracker coordinate system associated with the patient tracker 25. The same is true for the other positions, orientations and poses described herein as being defined in the tracking coordinate system.

Obtaining pose data may comprise obtaining tracking data indicative of a position and orientation of the tracker 22 attached to the medical imaging device. In this case, the current pose of the medical imaging device 10 may be determined based on the position and orientation of the tracker 22. Also, in this case, the tracker 22 preferably comprises three or more optical tracking markers 23.

Obtaining pose data may comprise obtaining one or more images of at least a predefined portion of the medical imaging device 10, acquired by the optical tracking system 24 or another sensing unit. In this case, the pose of the medical imaging device 10 may be determined by identifying and localizing the predefined portion in the one or more images, for example using a machine vision algorithm. This is because the localized pose of the predefined portion of the medical imaging device 10 is indicative of the current pose of the medical imaging device 10.

Obtaining pose data may comprise obtaining depth data depicting at least the predefined portion of the medical imaging device 10, the depth data being acquired by the optical tracking system 24 (e.g., a stereo camera thereof) or by another sensing unit. In this case, the pose of the medical imaging device 10 may be determined by localizing the predefined portion based on the depth data.

Obtaining pose data may comprise, at 204, obtaining first tracking data. The first tracking data is indicative of a plurality of first positions 46 of the tracker 22 attached to the medical imaging device 10. The plurality of first positions 46 correspond to locations of the tracker 22 at different points in time during a first movement of the medical imaging device 10 relative to the optical tracking system 24. As only positions of the tracker 22, no orientations thereof need to be obtained in this case, the tracker 22 may comprise only one optical tracking marker 23. The first movement may be conducted as part of a collision check, i.e., to check whether the medical imaging device 10 collides with persons or objects when moving in a predefined manner, preferably when moving similar as during a subsequent patient scan. The first movement may consist of a rotation of the medical imaging device 10, for example around its isocenter.

Obtaining pose data may comprise, at 206, determining the current pose of the medical imaging device 10 relative to the optical tracking system 24 based on the first tracking data, in particular based on the plurality of first positions 46 of the tracker 22.

Determining the current pose of the medical imaging device 10 relative to the optical tracking system 24 may comprise, at 208, determining a center of rotation 44 of the medical imaging device 10, and, optionally, determining a current position of the tracker 22, based on the plurality of first positions 46. The current pose of the medical imaging device 10 relative to the optical tracking system 24 may then be determined based on the center of rotation 44 of the medical imaging device 10 and, optionally, based on the current position of the tracker 22. The current pose of the medical imaging device 10 may be determined based on the center of rotation 44 and further based on (i) a current position and orientation of the tracker 22 and/or (ii) the localized predefined portion of the medical imaging device 10. Instead of the current position (and, optionally, the current orientation) of the tracker 22, a previous position (and, optionally, orientation) thereof may be used. This previous position (and, optionally, orientation) may correspond to a start position (and, optionally, start orientation) that the tracker has at a start of the first movement of the imaging unit 10, or correspond to an end position (and, optionally, end orientation) that the tracker has at a end of the first movement of the imaging unit 10. In this case, the first positions 46 may include the start position and/or the end position.

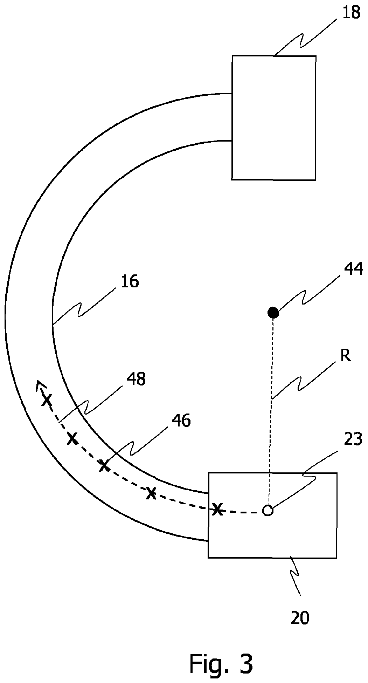

The approach of steps 204-208 is exemplarily shown in Fig. 3. As can be seen, the tracker 22 may consist of a single optical marker 23. In this case, only a three-dimensional position of the optical marker 23 may be able to be determined by the optical tracking system 24. In order to nevertheless derive a current pose of the medical imaging device 10 based on the localized tracker 22, the plurality of first positions of the tracker 22, as detected by the optical tracking system 24 during the first movement, can be used to derive a movement path 48 of the tracker 22. Under the assumption that this movement path 48 is a circle, the center 44 of this circle with radius R can be determined. This center 44 can be taken as the center of rotation of the medical imaging device 10, which, for example in the case of a C-arm, is also known as isocenter. The combination of the spatial position of the isocenter and the current spatial position of the tracker 22 yields the current pose of the medical imaging device 10 relative to the optical tracking system 24.

At 210, movement data is obtained. The movement data is indicative of a predicted movement that the medical imaging device 10 will undergo for acquiring a medical image and/or scan of the patient's body 12. The predicted movement may be defined in a coordinate system of the medical imaging device 10. The predicted movement may correspond to a movement of a movable portion of the medical imaging device 10, for example a movement of the X-ray source 18, the C-arm 16 and/or the detector 20. The predicted movement may comprise or consist of a translation and/or a rotation. In one variant, the predicted movement consists of a rotation around the isocenter.

At 212, a predicted movement path 42 is determined based on the pose data and the movement data obtained at 202, 210. The predicted movement path 42 is associated with the medical imaging device 10. This means that at least a predefined portion of the medical imaging device 10, a component (e.g., the tracker 22) attached to the predefined portion of the medical imaging device 10 and/or a point having a fixed spatial relationship to the predefined portion of the medical imaging device 10 follows the predicted movement path when the medical imaging device 10 moves for acquiring the medical image and/or scan of the patient's body 12. The predicted movement path may describe a path along which a tracker 22, mounted to the medical imaging device 10 and having a predefined relative pose thereto, moves when the medical imaging device 10 undergoes its predicted movement for acquiring a medical image of a patient. The predicted movement path 42 may be determined in the tracking coordinate system.

At 214, viewing data is obtained. The viewing data is indicative of a current field of view of the optical tracking system 24, in particular relative to the medical imaging device 10. The viewing data may define a spatial volume in which optical markers can be localized by the optical tracking system 24. This spatial volume may be defined in the tracking coordinate system.

At 216, based on the predicted movement path 42 and the viewing data, at least one first portion 46 of the predicted movement path 42 that extends through the field of view of the optical tracking system 24 and/or at least one second portion of the predicted movement path 42 that extends outside the field of view of the optical tracking system 24 is determined. Each of these portions may be defined in the tracking coordinate system.

At 218, object data is obtained. The object data is indicative of a current pose of an object arranged within the field of view of the medical imaging device 24, for example a current pose of a person 34 or of medical equipment such as the cart 36. The current pose of the object may be defined in the tracking coordinate system. The current pose of the object may be determined in a similar manner as the pose of the predefined portion of the medical imaging device 10. That is, the current pose of the object may be determined based on tracking data, one or more images and/or depth data, as described above for determining the current pose of the medical imaging device 10.

At 220, based on the predicted movement path and the object data, at least one third portion 50 of the predicted movement path 42 is determined that is hidden to the optical tracking system 24 by the object 34, 36. The at least one third portion 50 may be defined in the tracking coordinate system.

At 222, a representation of the one or more determined portion(s) is determined relative to the field of view of the optical tracking system 24. In other words, a representation of the one or more determined portions as seen from the perspective of the optical tracking system 24 is determined. The representation(s) may be determined in the tracking coordinate system. The representation may be determined such that it complies with a viewing direction of a camera 24, 26 or 28 of the optical tracking system 24. The representation may be determined as having an optical property that depends on a proportion between {i} a total size of all of the at least one first portion or a continuous section thereof and {ii} a total size of all of the at least one second portion or a continuous section thereof. For example, the optical property is selected from one of a predefined set of optical properties, each of the predefined set of optical properties being associated with a different range of the proportion. The predefined set of optical properties may consist of a plurality of different colors and/or a plurality of different patterns.

At 224, an image or video stream, acquired by the camera of the optical tracking system, is obtained.

At 226, based on at least the predicted movement path 42, display of a visualization is triggered. The visualization may be triggered to be displayed on the display 38 of the system 100. The visualization is configured to guide a user in repositioning the optical tracking system 24 and the medical imaging device 10 relative to one another such the medical imaging device 10 can be tracked via the optical tracking system 24 over at least a predefined minimum portion of the predicted movement path 42. Thereby, the visualization is configured to improve use of the medical imaging device 10 and the optical tracking system 24. The predefined minimum portion may be defined relative to the overall length of the predicted movement path and may correspond to, for example, 20, 25, 40, 50, 75 or 85 percent of length of the overall predicted movement path. Alternatively, the predefined minimum portion may be defined in absolute terms. In this case, the predefined minimum portion may be defined as an absolute length of movement (e.g., 30, 50, 80 or 100 cm) or as an absolute rotational value (e.g., 20, 25, 40, 50, 75 or 85 degrees of rotation). The visualization may be determined based on one or more of the determined portions of the predicted movement path 42 that extend through the field of view, extend outside the field of view and/or are hidden to the optical tracking system by the object 34, 36. The visualization may in particular include the representation(s) determined at 222. The visualization may comprise an overlay of the determined representation(s) over the image or video stream obtained at 224.

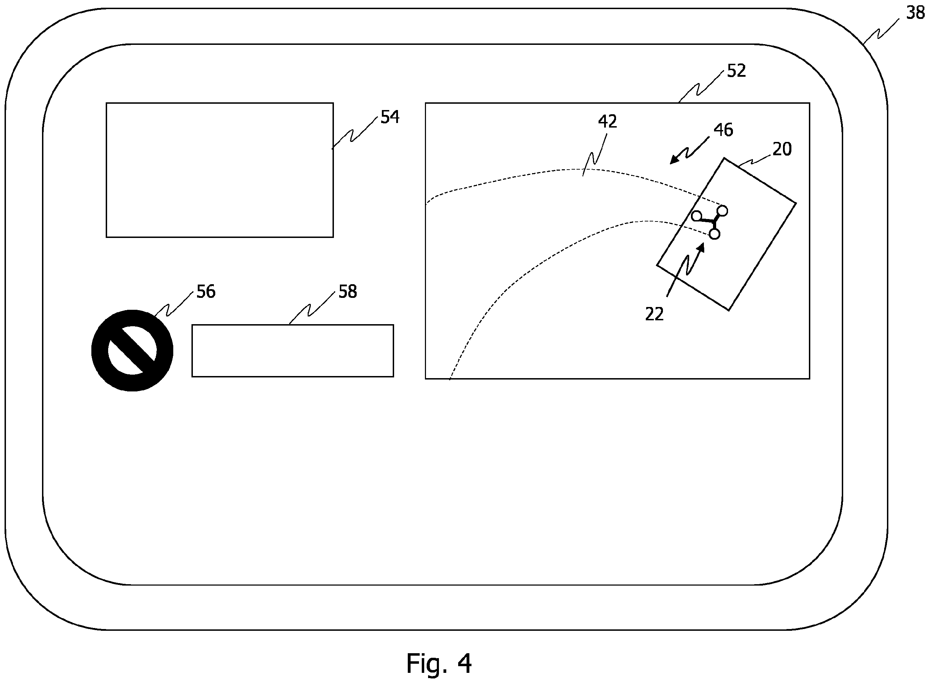

Exemplary visualizations that can be triggered to be displayed at 226 are shown in Figs. 4 to 6.

In the example of Fig. 4, only an unacceptably small portion of the predicted movement path 42 is visible to the optical tracking system 24. Here, the visualization includes a first portion 52 in which a representation of a first portion 46 of the predicted movement path 42 of the optical tracker 22 that extends within the field of view of the RGB camera 32 of the optical tacking unit 24 is shown. As this portion in the shown example is not large enough for a desired tracking of the medical imaging device 10, the representation of the first portion 46 may be colored red. This representation is overlaid over a video stream captured by the RGB camera 32 of the optical tracking system 24. In the illustrated example, the detector 20 having the tracker 22 attached thereto can be seen in the video stream. The visualization in Fig. 4 further includes a second portion 54 in which instructions for a user can be displayed. These instructions may explain to the user as text, image(s) and/or animation(s) how he should reposition the optical tracking system 24 relative to the medical imaging device 10 such that an acceptably large portion of the predicted movement path 42 will be visible to the optical tracking system 24. The visualization in Fig. 4 further includes a warning sign 56 and an explanation 58 as text, image(s) and/or animation(s) that explain to the user what the problem is, in particular that only an unacceptably small portion of the predicted movement path 42 is visible to the optical tracking system 24 in the current pose. Of course, it is possible to omit some of these displayed elements or to arrange the same at other locations in the visualization on the display 38.

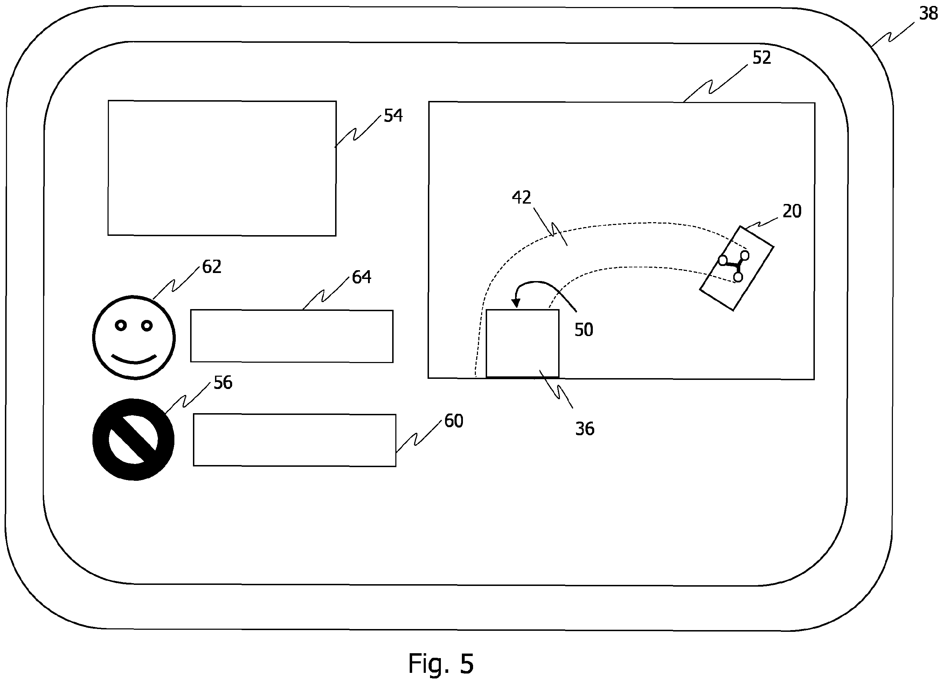

In the example of Fig. 5, a third portion 50 of the predicted movement path 42 is hidden from the optical tracking system 24, so the remaining visible part of the predicted movement path 42 that lies within the field of view of the optical tracking system 24 is unacceptably small. As the visible portion of the predicted movement path 42 is not large enough for a desired tracking of the medical imaging device 10 due to the unwanted covering by the object 36, the representation of this portion may be colored blue. This representation is overlaid over a video stream captured by the RGB camera 32 of the optical tracking system 24. In the illustrated example, the detector 20 having the tracker 22 attached thereto can be seen in the video stream. Here, the object 36 that hides part of the predicted movement path 42 from the optical tracking system 24 is also shown. The visualization in Fig. 5 further includes a second portion 54 in which instructions for a user can be displayed. These instructions may explain to the user as text, image(s) and/or animation(s) how he should reposition the optical tracking system 24 relative to the medical imaging device 10 such that an acceptably large portion of the predicted movement path 42 will be visible to the optical tracking system 24. This may comprise an instruction to move the object 36. The visualization in Fig. 4 further includes a warning sign 56 and an explanation 60 as text, image(s) and/or animation(s) that explain to the user what the problem is, in particular that only an unacceptably small portion of the predicted movement path 42 is visible to the optical tracking system 24 in the current pose due to the object 36 covering a portion 50 of the otherwise observable predicted movement path 42. The visualization in Fig. 5 further includes a positive icon 62 and an explanation 64 as text, image(s) and/or animation(s) that explain to the user that the first portion of the predicted movement path is large enough, i.e., that an acceptably large portion of the predicted movement path 42 lies within the field of view of the optical tracking system 24. Also here, it is possible to omit some of these displayed elements or to arrange the same at other locations in the visualization on the display 38.

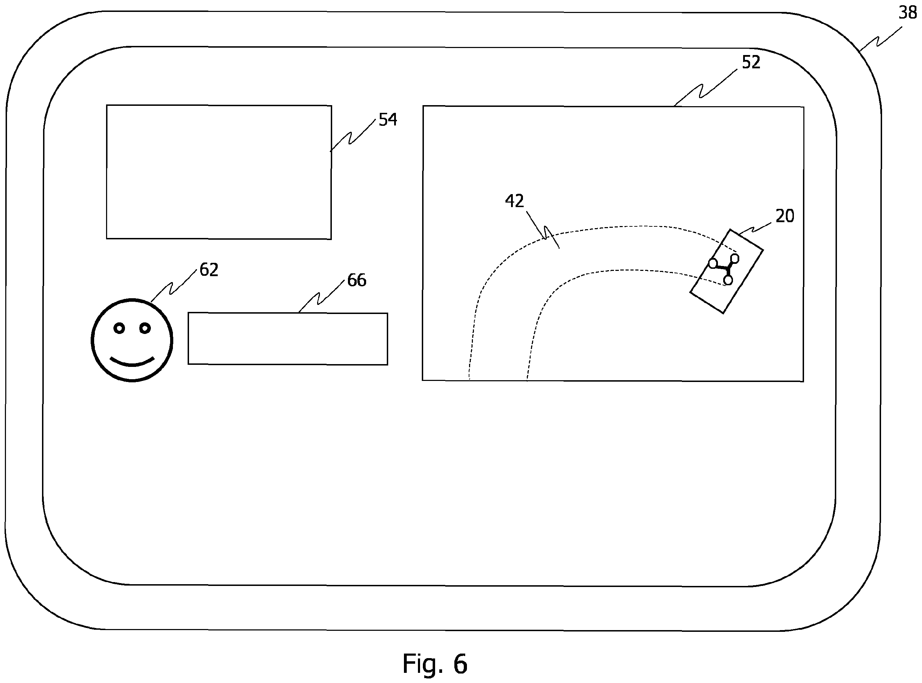

In the example of Fig. 6, an acceptably large portion of the predicted movement path 42 is visible to the optical tracking system 24. Here, the visualization includes a first portion 52 in which a representation of the first portion 46 of the predicted movement path 42 of the optical tracker 22 that extends within the field of view of the RGB camera 32 of the optical tacking unit 24 is shown. As this portion in the shown example is large enough for a desired tracking of the medical imaging device 10, the representation of the first portion 46 may be colored green. This representation is overlaid over a video stream captured by the RGB camera 32 of the optical tracking system 24. In the illustrated example, the detector 20 having the tracker 22 attached thereto can be seen in the video stream. The visualization in Fig. 6 further includes a second portion 54 in which instructions for a user can be displayed. These instructions may explain to the user as text, image(s) and/or animation(s) how he may reposition the optical tracking system 24 relative to the medical imaging device 10 such that an even larger portion of the predicted movement path 42 will be visible to the optical tracking system 24. These instructions may inform the user how to proceed for acquiring a patient image or patient scan. The visualization in Fig. 6 further includes a positive icon 62 and an explanation 66 as text, image(s) and/or animation(s) that explain to the user that an acceptably large portion of the predicted movement path 42 can be observed by the optical tracking system 24, so the system 100 is now in condition to acquire a medical image or a CT scan of the patient's body 12.

As indicated with dashed arrow 228, the visualization may be updated in case the pose of the medical imaging device 10 relative to the optical tracking system 24 changes. In other words, updated pose data may be obtained, which is indicative of a repositioned pose of the medical imaging device 10 relative to the optical tracking system 24, the predicted movement path 42 may then be updated based on the updated pose data, and based on the updated predicted movement path, the visualization may be updated accordingly. In this case, one may say that the visualization provides a real-time guidance for a user for repositioning the optical tracking system 24 and the medical imaging device 10 relative to one another.

At 230, second tracking data is obtained. The second tracking data is indicative of a plurality of second positions of the tracker 22, wherein each of the second positions is associated with a different point in time during a second movement of the medical imaging device 10 relative to the optical tracking system 24. The second movement may occur for acquiring a medical image and/or scan of the patient's body 12. That is, during the second movement, a medical patient scan such as a CT scan may be acquired by the medical imaging device 10.

At 232, based on the plurality of second positions, a center of rotation 44 of the medical imaging device 10 is determined. This determination may be performed in a similar manner as described above for the plurality of first positions, see in particular the above explanation of steps 204, 206, 208.

At 234, medical image data associated with the second movement of the medical imaging device 10 is registered, based on the center of rotation 44 of the medical imaging device determined at 232. The medical image data preferably comprises a patient scan acquired by the medical imaging device 10 during the second movement, and/or a medical image acquired during or after the second movement of the medical imaging device 10. Registering the medical image data may comprise determining a transformation between an image coordinate system and the tracking coordinate system. The image coordinate system may have a predefined spatial relationship to the center of rotation 44.

It is also possible to acquire a medical image, via the medical imaging device 10, of a calibration device that comprises X-ray-opaque fiducials and optical tracking markers. The calibration device may then be localized both in the medical image and by the optical tracking system 24, to thereby determine a transformation between the image coordinate system and the tracking coordinate system. This transformation may then be used to register the medical image data. Other ways of registering medical image data are also possible.

At 236, the registered medical image data is used to provide a surgical navigation view for a user. This may comprise determining and triggering display of said navigation view on the display 38. For example, the surgical navigation view includes a representation of a body part of the patient's body 12 as depicted in the medical image data. The navigation view may also include an indication of a surgical instrument that is tracked by the optical tracking system 24.

The technique disclosed herein will now be explained in other words.

As explained above, the proposed technique is related to the field of surgical navigation. The stereo camera 26, 28 can be used to localize instruments, the patient and other objects, using optical trackers (e.g., 22, 25) attached thereto. Prior or during the clinical procedure, medical (e.g., X-ray) images of the patient's body 12 can be acquired. Those images can be registered to the patient's body 12 using a so-called "image registration step", which allows to determine the relationship between the medical images and the patient tracker 25 affixed to the patient's body 12. Thus, the surgeon can move trackable instruments on the physical anatomy while the navigation system overlays a representation of these tracked instruments on the medical images in a navigation view. Multiple imaging modalities can be used, such as magnetic resonance imaging (MRI), computed tomography (CT) or Cone Beam Computed Tomography (CBCT). As shown in Fig. 1, the medical imaging device 10 used for acquiring medical images can comprise a C-arm 16, which can rotate around the patient's body 12 to generate X-ray-based images, for example as part of a CT or CBCT scan.

The medical imaging device 10 may be configured as a so-called 3D C-arms, and provide a 3-dimensional representation of the patient's body 12 by taking a large number of fluoroscopy shots while spinning around the patient's body 12. From these images, a 3D representation of the patient can be computed, also known as "image volume". To enable for this reconstruction with a required accuracy, the C-arm should move on a pre-defined trajectory.

Several image registration techniques exist, which often involve a manual process and can be time-consuming, especially when the images were acquired at a time before surgery. In certain procedures, the imaging device unit is available during the surgery, so intra-operative imaging is possible. In such workflows, it is possible to seamlessly integrate the imaging step with the navigation and utilize automatic image registration workflows. Such automatic registration workflows rely on the fact that the images acquired by the medical imaging device 10 will always be located in the same position and orientation with respect to the C-arm 16. A tracker 22 should be affixed to the imaging device 10, as illustrated in Fig. 1. A calibration step, called "C-arm calibration", can be performed, which allows to determine the transformation between the tracker 22 and the image volume, in other words, a transformation between the tracking coordinate system of the optical tracking system 224 and the image coordinate system, which typically has a fixed pose relative to the C-arm 16. Such a C-arm calibration can account for mechanical deviations in the overall imaging system which may happen over time and which may negatively impact navigational accuracy. The calibration can be performed in a static reference position, such as a start position present before a scan is started.

During the clinical procedure, an intra-operative scan of the patient can be acquired as medical image data. The C-arm tracker 22 can be used to determine the pose of the C-arm 16, in the same static reference position in which the medical imaging device 10 was calibrated (e.g. the start position before the scan). As the calibration transformation is known, the location of the medical images (e.g., in DICOM format) comprised in the medical image data with respect to the C-arm tracker 22 is known. This in turn allows to automatically determine the registration transformation, in particular the spatial relationship between the patient tracker 22 and the medical images. The registration transformation can define a transformation between the tracking coordinate system and the image coordinate system. During a clinical case, the tracker 22 may only be used in the reference position where it was calibrated, and it may not be tracked during the scan.

After each procedure, the C-arm tracker 22 may be detached for reprocessing purposes. This means that the physical tracker 22 used for registration can be a different one than the physical tracker which was used for C-arm calibration. Additionally, one may assume that the tracker 22 does not move once it is mounted on the medical imaging device 10. Consequently, small errors in the tracker position and orientation, introduced at the level of the mechanical attachment interface, can lead to large errors at the image-level. This effect is particularly true for C-arms, as there is a large distance between the C-arm tracker 22 and the image, typically above 500mm. Using basic trigonometry calculations, one can calculate an order of magnitude: rotational errors as small as 0.25° can lead to errors superior to 2mm, at a distance of 500mm. Depending on the use case, a 2mm error may represent a significant safety issue for the patient, e.g. when the surgeon is operating near critical anatomy such as the brain or the spinal cord.

In order to minimize the mechanical positioning variability, the following strategies may be implemented:
a) Minimize mechanical play. This however can only be done to a certain extent: if the mechanical play is too small, it will become difficult for the surgical staff to mount the tracker 22, resulting in user annoyance.
b) Increase the number of tracker fixation points and their relative distance. This depends on the interface the imaging device manufacturer provides.
c) Increase the size of the tracker attachment interface. The larger the guiding elements thereof, the better the positional repeatability. This has the same limitations as strategy a).

These strategies may result in a bulky tracker attachment interface on the medical imaging unit 10, which represents an increased risk of collision with objects in the surroundings of the imaging unit 10. The operating room (OR) is typically a crowded environment, and surgeons generally favor compact devices. This also can lead to an increased mounting time of the tracker 22, while procedure duration is also a factor which should be minimized.

In optical tracking such as used in surgical navigation, the accuracy can be broken down into trueness and precision. When repeating the same measurement multiple times, precision describes how close together the measured points are, while trueness describes how close the mean of the point cloud is to the actual measured object. Precision reflects the randomness of the measurements, while trueness represents a systematic bias. If this concept is applied to optical tracking, for a given distance between the optical tracker and a Tool Center Point (TCP) of a tracked and navigated instrument, a larger tracker will result in an improved precision and trueness. Generally, both trueness and precision are important, but for C-arm tracking, trueness is deemed to be most relevant. As explained above, the imaging device 10 may only be tracked in a static reference position. Thus, multiple measurements can be taken, and an average can be calculated to determine the pose of the imaging device 10. As the data is averaged, the impact of precision is less relevant with longer averaging duration under certain assumptions. However, the averaging duration would not correct for any trueness errors. Trueness errors in optical tracking can arise from multiple sources, such as variability in tracker manufacturing. The trueness errors then might have an impact on the accuracy and introduce significant errors. Although large trackers are advantageous in this respect, surgeons generally favor compact devices.

One aspect of the technique disclosed herein leverages the repeatable trajectory of motion the C-arm 16 performs during a 3D scan, to overcome the two previously mentioned issues. This aspect is applicable to calibration and registration workflows alike and generally comprises the following parts (1) to (3).

### (1) Measurement of C-arm rotation

In this part, the optical tracking system 24 captures the plurality of positions of the C-arm tracker 22 for the duration of scanner rotation. This also includes the static position before the scan starts.

### (2) Circle fit

The acquired positions of the tracker 22 can be fitted to a circle with radius R around circle center 44, as shown in Fig. 3.

### (3) Determination of pose of imaging unit 10

Based on the center 44 of the fitted circle and the start position of the C-arm movement, the pose of the medical imaging unit 10 can be determined.

The determined pose of the imaging unit 10 can then be used to reference the image volume. This effectively solves the two issues described in the previous section.

Instead of using an optical tracker with at least three fiducials to obtain the plurality of positions, one may use only a single optical marker 12 to track the imaging device 10. Normally, with only a single marker 23 tracked by the optical tracking system 24, only its position can be determined, but orientation cannot. By implementing the aspect described above, only the plurality of positions of the tracker 22 and not the orientation thereof are required when the marker 23 is moved on a known movement path, e.g. a circle. The single marker 23 can be active, i.e. a LED, or passive, e.g. a stick-on fiducial.

An added-value of this approach is that is does not required any additional workflow steps. Usually, using flat stick-on fiducials requires an additional step of acquiring the geometry of the so-created tracker 22, thereby creating a so-called "rigid body" consisting of multiple flat-fiducials. Here, as only on the positions of the single-tracker, such approach is not needed. It is also worth noting the advantage in terms of usability of only having to stick a fiducial on a scanner to allow automatic image registration.

Generally, more accurate results are obtained the more parts of the movement path of the tracker 22 can be recorded via the optical tracking system 24 during the scan of the imaging unit 10. To this end, the surgical navigation system 100 may comprise the Q-Guidance Cart of Stryker^{®} with a FP8000 camera as optical tracking system 24, which offers a sufficient field of view. As the tracker 22 will move with the C-arm 16 during scanning, it may be challenging for the user to adjust the tracking system 24 in a way that the C-arm tracker 22 is visible in large parts of the movement path, preferably including the start position of the C-arm 16 and the end position. On the other hand, the whole movement path may not need to be recorded, but the more portions are available, the more reliable becomes the determined pose of imaging unit 10, in particular when using the center of rotation 44 as a basis (e.g., based on the circle fit approach explained above).

To offer guidance to the user, a Live Cam view may be displayed on display 38 with an overlay to illustrate the predicted movement path 42 of the tracker 22 and/or at least a part of the C-arm 16. This overlay can be scaled and adjusted to the Live Cam view by leveraging different methods to identify the C-arm's position and orientation, for example by employing machine vision, a depth map or recording the tracker movement during a collision check prior to the actual scan. Examples of such Live Cam views are illustrated at reference sign 52 in Figs. 4, 5 and 6.

The color of the overlay can be adjusted according to percentage of visible rotation throughout the scan, giving an indication to the user about the estimated achievable accuracy. For example, the overlay could turn green when more than 50% of the overall rotation is visible, and red if less than 30% of the rotation is visible.

To do so, a prediction algorithm may be used to compute this percentage. The dimensions of the C-arm 16 and its rotation trajectory may be predefined. These can be computed, in the C-arm tracker reference frame when it is in the initial starting position. One then has a set of points, expressed in C-arm tracker coordinates, which correspond to the predicted rotation. Those points can then be transformed into the tracking coordinate system, by applying the transformation between the tracking system 24 and the C-arm tracker 22. Additionally, the camera working volume, also referred to as field of view, is known. For any point expressed in the camera coordinate system, it is known if it is inside the camera volume or not. Such a check can be applied to all the points of the predicted rotation. One can thus determine the percentage of visible rotation throughout the scan.

For the above approach, it is preferred for the movement of the C-arm during scans to be repeatable and accurate. Otherwise, the C-arm internal reconstruction of the 3D representation of the patient may be less accurate and auto-registration methods for C-arms that rely on a repeatable start position may provide inaccurate registrations. As explained herein, user guidance is provided to support the user in adjusting the pose of the tracking system 24 relative to the imaging device 10 to record as much as possible of the C-arm motion trajectory. An algorithm can estimate the percentage of visible rotation and the user can be informed accordingly.

With respect to the issues as mentioned above, the technique disclosed herein may provide the following advantages:

### Issue 1: Errors related to mechanical attachment interface for the tracker 22

Mounting variability, which can introduce rotational error, is less relevant for the proposed solution as far as it only relies on position measurement. Systematic errors may be corrected by averaging over the trajectory of motion of the C-arm. Preferably, the tracker 22 remains rigidly in place after mounting. The position of the single marker 23 may not change between calibration and registration workflow, but is generally free to place on the imaging device 10. Accordingly, the technique disclosed herein improves use of the imaging device 10 and the tracking system 24.

### Issue 2: Size of tracker due to large tracker-TCP distance

Any trueness error at the tracker-level is less relevant in case systematic errors are corrected by considering the range of motion of the C-arm 16. In addition, when using the center of rotation 44 for computing the pose of the medical imaging unit 10, the distance to the TCP can be reduced to approximately 160mm compared with approaches where the pose of the tracker 22 in the start position of the C-arm 16 is used as sole input to compute the transform to the image, in which approaches a long TCP distance of approx. 500mm is present. Hence, the approach described herein may be less prone to angular errors. Eventually, the technique works with the smallest tracker 22 possible, that consists of a single optical marker 23, which does not limit the working space in the operating room.

As understood herein, the technique disclosed herein, including the visualization that is triggered to be displayed, the particular determination of the current pose of the imaging device 10 (e.g., using the center of rotation 44), the image registration and/or the subsequent navigation view, improves use of the imaging device 10 and the tracking system 24. Thus, the technique is also referred to as a technique for improving use of a medical imaging device and an optical tracking system.

Various modifications of the present technique are possible. The examples, aspects, approaches, the method and the system disclosed with reference to the drawings may be supplemented with features as discussed for the "aspects" in the "Summary" portion of the description, and vice versa.

## Claims

1. A method for improving use of a medical imaging device and an optical tracking system, the method being performed by at least one processor and comprising:
obtaining pose data indicative of a current pose of a medical imaging device relative to an optical tracking system;
obtaining movement data indicative of a predicted movement that the medical imaging device will undergo for acquiring a medical image of a patient;
determining, based on the pose data and the movement data, and relative to the optical tracking system, a predicted movement path that is associated with the medical imaging device; and
based on the predicted movement path, triggering display of a visualization that is configured to guide a user in repositioning the optical tracking system and the medical imaging device relative to one another such the medical imaging device can be tracked via the optical tracking system over at least a predefined minimum portion of the predicted movement path.

2. The method of claim 1, further comprising:
obtaining viewing data indicative of a current field of view of the optical tracking system; and
determining, based on the predicted movement path and the viewing data, at least one first portion of the predicted movement path that extends through the field of view of the optical tracking system and/or at least one second portion of the predicted movement path that extends outside the field of view of the optical tracking system,
wherein the visualization is determined based on one or more of said determined portion(s) of the predicted movement path.

3. The method of claim 2, further comprising:
obtaining object data indicative of a current pose of an object arranged within the field of view of the medical imaging device; and
determining, based on the predicted movement path and the object data, at least one third portion of the predicted movement path that is hidden to the optical tracking system by the object,
wherein the visualization is determined based on one or more of said determined portion(s) of the predicted movement path.

4. The method of claim 2 or 3, further comprising:
determining a representation of said one or more determined portion(s) relative to the field of view of the optical tracking system,
wherein the visualization includes said representation.

5. The method of claim 4, wherein the representation is determined such that it complies with a viewing direction of a camera of the optical tracking system.

6. The method of claim 4 or 5, wherein the representation is determined as having an optical property that depends on a proportion between {i} a total size of all of the at least one first portion or a continuous section thereof and {ii} a total size of all of the at least one second portion or a continuous section thereof.

7. The method of claim 6, wherein the optical property is selected from one of a predefined set of optical properties, each of the predefined set of optical properties being associated with a different range of the proportion.

8. The method of claim 7, wherein the predefined set of optical properties consists of a plurality of different colors and/or a plurality of different patterns.

9. The method of at least claim 5, further comprising:
obtaining an image or video stream acquired by the camera of the optical tracking system,
wherein the visualization comprises an overlay of the determined representation over the image or video stream.

10. The method of any one of claims 1 to 9, wherein the pose data is indicative of a pose of a tracker that is mounted to the medical imaging device and has a predefined relative pose thereto.

11. The method of any one of claims 1 to 10, wherein the predicted movement path describes a path along which a tracker, mounted to the medical imaging device and having a predefined relative pose thereto, moves when the medical imaging device undergoes its predicted movement for acquiring a medical image of a patient.

12. The method of claim 10 or 11, wherein the tracker comprises a single passive optical tracking marker.

13. The method of any one of claims 10 to 12, further comprising:
obtaining first tracking data indicative of a plurality of first positions of the tracker at different points in time during a first movement of the medical imaging device relative to the optical tracking system; and
based on the plurality of first positions, determining the current pose of the medical imaging device relative to the optical tracking system to thereby obtain the pose data.

14. The method of claim 13, wherein, based on the plurality of first positions, a center of rotation of the medical imaging device and a current position of the tracker are determined, and the current pose of the medical imaging device relative to the optical tracking system is determined based on the center of rotation of the medical imaging device and the current position of the tracker.

15. The method of any one of claims 1 to 14, further comprising:
obtaining updated pose data indicative of a repositioned pose of the medical imaging device relative to the optical tracking system;
updating the predicted movement path based on the updated pose data; and
based on the updated predicted movement path, updating the visualization that is triggered to be displayed.

16. The method of at least claim 13, further comprising:
obtaining second tracking data indicative of a plurality of second positions of the tracker at different points in time during a second movement of the medical imaging device, for acquiring a medical image, relative to the optical tracking system
based on the plurality of second positions, determining a center of rotation of the medical imaging device; and
registering medical image data associated with the second movement of the medical imaging device, based on the determined center of rotation-of the medical imaging device.

17. The method of claim 16, further comprising:
using the registered medical image data to provide a surgical navigation view for a user.

18. A surgical navigation system comprising at least one processor, the at least one processor being configured to perform the method of any one of claims 1 to 17.

19. The surgical navigation system of claim 18, further comprising at least one of the following entities:
a display configured to display the visualization;
the optical tracking system;
the medical imaging device;
the tracker.

20. A computer program comprising instructions which, when executed by at least one processor, cause the at least one processor to perform the method of any one of claims 1 to 17, wherein the computer program is optionally carried by at least one carrier such as a data stream, a memory or a non-transitory computer storage medium.

## Amended claims

### Amended claims in accordance with Rule 137(2) EPC.

1. A method for improving use of a medical imaging device (10) and an optical tracking system (24), the method being performed by at least one processor (2) and comprising:
obtaining pose data (202) indicative of a current pose of a medical imaging device (10) relative to an optical tracking system (24);
obtaining movement data (210) indicative of a predicted movement that the medical imaging device (10) will undergo for acquiring a medical image of a patient (12);
determining (212), based on the pose data and the movement data, and relative to the optical tracking system (24), a predicted movement path (42) that is associated with the medical imaging device (10); and
based on the predicted movement path (42), triggering (226) display of a visualization that is configured to guide a user in repositioning the optical tracking system (24) and the medical imaging device (10) relative to one another such the medical imaging device (10) can be tracked via the optical tracking system (24) over at least a predefined minimum portion of the predicted movement path (42).

2. The method of claim 1, further comprising:
obtaining viewing data (214) indicative of a current field of view of the optical tracking system (24); and
determining (216), based on the predicted movement path (42) and the viewing data, at least one first portion of the predicted movement path (42) that extends through the field of view of the optical tracking system (24) and/or at least one second portion of the predicted movement path (42) that extends outside the field of view of the optical tracking system (24),
wherein the visualization is determined based on one or more of said determined portion(s) of the predicted movement path (42).

3. The method of claim 2, further comprising:
obtaining object data (218) indicative of a current pose of an object arranged within the field of view of the medical imaging device (10); and
determining (220), based on the predicted movement path (42) and the object data, at least one third portion of the predicted movement path (42) that is hidden to the optical tracking system (24) by the object,
wherein the visualization is determined based on one or more of said determined portion(s) of the predicted movement path (42).

4. The method of claim 2 or 3, further comprising:
determining (222) a representation of said one or more determined portion(s) relative to the field of view of the optical tracking system (24),
wherein the visualization includes said representation.

5. The method of claim 4, wherein the representation is determined such that it complies with a viewing direction of a camera (32) of the optical tracking system (24).

6. The method of claim 4 or 5, wherein the representation is determined as having an optical property that depends on a proportion between {i} a total size of all of the at least one first portion or a continuous section thereof and {ii} a total size of all of the at least one second portion or a continuous section thereof.

7. The method of claim 6, wherein the optical property is selected from one of a predefined set of optical properties, each of the predefined set of optical properties being associated with a different range of the proportion.

8. The method of claim 7, wherein the predefined set of optical properties consists of a plurality of different colors and/or a plurality of different patterns.

9. The method of at least claim 5, further comprising:
obtaining (224) an image or video stream acquired by the camera (32) of the optical tracking system (24),
wherein the visualization comprises an overlay of the determined representation over the image or video stream.

10. The method of any one of claims 1 to 9, wherein the pose data is indicative of a pose of a tracker that is mounted to the medical imaging device (10) and has a predefined relative pose thereto.

11. The method of any one of claims 1 to 10, wherein the predicted movement path (42) describes a path along which a tracker (22), mounted to the medical imaging device (10) and having a predefined relative pose thereto, moves when the medical imaging device (10) undergoes its predicted movement for acquiring a medical image of a patient (12).

12. The method of claim 10 or 11, wherein the tracker comprises a single passive optical tracking marker (23).

13. The method of any one of claims 10 to 12, further comprising:
obtaining (204) first tracking data indicative of a plurality of first positions of the tracker at different points in time during a first movement of the medical imaging device (10) relative to the optical tracking system (24); and
based on the plurality of first positions, determining the current pose of the medical imaging device (10) relative to the optical tracking system (24) to thereby obtain the pose data.

14. The method of claim 13, wherein, based on the plurality of first positions, a center of rotation of the medical imaging device (10) and a current position of the tracker are determined, and the current pose of the medical imaging device (10) relative to the optical tracking system (24) is determined based on the center of rotation of the medical imaging device (10) and the current position of the tracker.

15. The method of any one of claims 1 to 14, further comprising:
obtaining (206) updated pose data indicative of a repositioned pose of the medical imaging device (10) relative to the optical tracking system (24);
updating (228) the predicted movement path (42) based on the updated pose data; and
based on the updated predicted movement path (42), updating the visualization that is triggered to be displayed.

16. The method of at least claim 13, further comprising:
obtaining second tracking data (230) indicative of a plurality of second positions of the tracker at different points in time during a second movement of the medical imaging device (10), for acquiring a medical image, relative to the optical tracking system (24);
based on the plurality of second positions, determining (232) a center of rotation of the medical imaging device (10); and
registering (234) medical image data associated with the second movement of the medical imaging device (10), based on the determined center of rotation-of the medical imaging device (10).

17. The method of claim 16, further comprising:
using (236) the registered medical image data to provide a surgical navigation view for a user.

18. A surgical navigation system (100) comprising at least one processor (2), the at least one processor (2) being configured to perform the method of any one of claims 1 to 17.

19. The surgical navigation system of claim 18, further comprising at least one of the following entities:
a display (38) configured to display the visualization;
the optical tracking system (24);
the medical imaging device (10);
the tracker (22).

20. A computer program comprising instructions which, when executed by at least one processor, cause the at least one processor to perform the method of any one of claims 1 to 17, wherein the computer program is optionally carried by at least one carrier such as a data stream, a memory or a non-transitory computer storage medium.
